# EUROPÄISCHE PATENTANMELDUNG

(11) **EP 3 437 626 A1**
(43) Veröffentlichungstag der Anmeldung: **06.02.2019**
(21) Anmeldenummer: 18181897.2
(22) Anmeldetag: 05.07.2018
(51) Int. Cl.: A61K 8/35, A61K 8/49, A61Q 17/04

(54) **SONNENSCHUTZMITTEL MIT GERINGEN AUGENREIZUNGSPOTENTIAL**

(30) Priorität: 01.08.2017 DE 102017213219
(71) Anmelder: Beiersdorf AG, 20253 Hamburg (DE)
(72) Erfinder: Eitrich, Anja, 25337 Kölln-Reisiek (DE); Deinhard, Sybil-Marie, 22307 Hamburg (DE); Schade, Juliane, 22529 Hamburg (DE)

(57) **Zusammenfassung**

Verwendung einer UV-Filterkombination enthaltend 4-(tert.-Butyl)-4'-methoxydibenzoylmethan und 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) in kosmetischen Sonnenschutzmitteln, zur Verhinderung von Augenirritationen, wobei das Sonnenschutzmittel frei ist von 3-(4-Methylbenzyliden)-campher, 2-Hydroxy-4-methoxybenzophenon (INCI: Oxybenzon), Ethylhexyl-2-cyano-3,3-diphenylacrylat (INCI: Octocrylen) und 4-Methoxyzimtsäure-2-ethylhexylester (INCI Octylmethoxycinnamate), sowie ein Kosmetikum, welches ein solches Sonnenschutzmittel enthält.

## Beschreibung

Die vorliegende Erfindung betrifft die Verwendung einer UV-Filterkombination enthaltend 4-(tert.-Butyl)-4'-methoxydibenzoylmethan und 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) in kosmetischen Sonnenschutzmitteln, zur Verhinderung von Augenirritationen, wobei das Sonnenschutzmittel frei ist von 3-(4-Methylbenzyliden)-campher, 2-Hydroxy-4-methoxybenzophenon (INCI: Oxybenzon), Ethylhexyl-2-cyano-3,3-diphenylacrylat (INCI: Octocrylen) und 4-Methoxyzimtsäure-2-ethylhexylester (INCI Octylmethoxycinnamate), sowie ein Kosmetikum, welches ein solches Sonnenschutzmittel enthält.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Sonnenschutzmittel müssen üblicherweise neben dem Schutz vor UV-B Strahlung auch einen ausreichenden Schutz vor UV-A Strahlung aufweisen. Zu diesem Zwecke enthalten sie entsprechende UV-A-Filter, insbesondere 4-(tert.-Butyl)-4'-methoxydibenzoylmethan. Von 4-(tert.-Butyl)-4'-methoxydibenzoylmethan ist allgemein bekannt, dass dieser UV-A-Filter selbst nicht besonders fotostabil ist und daher mit nur in Kombination mit weiteren UV-Filtern eingesetzt werden kann. Für die Fotostabilisierung von 4-(tert.-Butyl)-4'-methoxydibenzoylmethan werden üblicherweise Ethylhexyl-2-cyano-3,3-diphenylacrylat (INCI: Octocrylen), 3-(4-Methylbenzyliden)-campher und/oder 4-Methoxyzimtsäure-2-ethylhexylester (INCI Octylmethoxycinnamate) eingesetzt. Diese Stoffe haben gegenüber dem ebenfalls zur Fotostabilisierung geeigneten 2,4-Bis-{[4-(2-ethyl-hexyloxy)-2-hydroxy]-phenyl}-6-(4-methoxyphenyl)-1,3,5-triazin (INCI: Bis- Ethylhexy-loxyphenol methoxyphenyl Triazine) den Vorteil, als bei Raumtemperatur flüssige Öle vorzuliegen, die das bei Raumtemperatur als Feststoff vorliegenden 4-(tert.-Butyl)-4'-methoxydibenzoylmethan zusätzlich lösen. Bis-Ethylhexy-loxyphenol methoxyphenyl Triazine hingegen ist ein ebenfalls relativ schwer löslicher Feststoff, der wie das 4-(tert.-Butyl)-4'-methoxydibenzoylmethan leicht aus den fertigen Zubereitungen wieder auskristallisiert. Die Temperatur- und Lagerstabilität ist also geringer als bei den UV-Filtern auf Ölbasis.

Nachteilig am Stande der Technik ist der Umstand, dass herkömmliche Sonnenschutzmittel mit 4-(tert.-Butyl)-4'-methoxydibenzoylmethan beim Auftragen auf die Gesichtshaut dazu neigen, durch "Zerfließen" auf der Haut in Kontakt mit dem Auge gelangen. Die Fachleute sprechen dann davon dass das Sonnenschutzmittel in das Auge einfließt. Im Auge selbst führen die Zubereitungen dann zu Augenirritationen (Augenreizungen, Augenrötungen, verschleierter Blick etc.), da die Zubereitungen ein nicht unerhebliches Augenreizungspotential aufweisen.

Es war daher die Aufgabe der vorliegenden Erfindung, ein stabiles Sonnenschutzmittel zu entwickeln, welches, beim Auftragen auf die Gesichtshaut bzw. beim Kontakt mit den Augen, zu deutlich weniger Augenirritationen (Augenreizungen, Augenrötungen, verschleierter Blick etc.) führt.

Gelöst wird die Aufgabe durch die Verwendung einer UV-Filterkombination enthaltend 4-(tert.-Butyl)-4'-methoxydibenzoylmethan und 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) in kosmetischen Sonnenschutzmitteln, zur Verhinderung von Augenirritationen, wobei das Sonnenschutzmittel frei ist von 3-(4-Methylbenzyliden)-campher, 2-Hydroxy-4-methoxybenzophenon (INCI: Oxybenzon), Ethylhexyl-2-cyano-3,3-diphenylacrylat (INCI: Octocrylen) und 4-Methoxyzimtsäure-2-ethylhexylester (INCI Octylmethoxycinnamate).

Gelöst wird die Aufgabe ferner durch die Verwendung einer UV-Filterkombination enthaltend 4-(tert.-Butyl)-4'-methoxydibenzoylmethan und 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) in kosmetischen Sonnenschutzmitteln, um das Einfließen desselben ins Auge bei der Anwendung im Gesicht zu verhindern, wobei das Sonnenschutzmittel frei ist von 3-(4-Methylbenzyliden)-campher, 2-Hydroxy-4-methoxybenzophenon (INCI: Oxybenzon), Ethylhexyl-2-cyano-3,3-di-phenylacrylat (INCI: Octocrylen) und 4-Methoxyzimtsäure-2-ethylhexylester (INCI Octylmethoxycinnamate).

Gelöst wird die Aufgabe nicht zuletzt durch ein Kosmetikum aus
a) einem Sonnenschutzmittel enthaltend 4-(tert.-Butyl)-4'-methoxydibenzoylmethan und 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine), wobei das Sonnenschutzmittel frei ist von 3-(4-Methylbenzyliden)-campher, 2-Hydroxy-4-methoxybenzophenon (INCI: Oxybenzon), Ethylhexyl-2-cyano-3,3-diphenylacrylat (INCI: Octocrylen) und 4-Methoxyzimtsäure-2-ethylhexylester (INCI Octylmethoxycinnamate), und
b) einem Packmittel, wobei das Packmittel einen schriftlichen Hinweis darauf enthält, dass das Sonnenschutzmittel ein nur geringes Augenreizungspotential aufweist.

Zwar kennt der Fachmann grundsätzlich verschiedene Sonnenschutzmittel, die neben 4-(tert.-Butyl)-4'-methoxydibenzoylmethan auch 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) enthalten, doch war über das Potential im Hinblick auf Augenirritationen von UV-Filterkombinationen mit 4-(tert.-Butyl)-4'-methoxydibenzoylmethan wenig bekannt.

Die Formulierungen "erfindungsgemäß", "erfindungsgemäß vorteilhaft", "vorteilhaft im Sinne der Vorliegenden Erfindung" etc. beziehen sich im Rahmen der vorliegenden Offenbarung immer sowohl auf die erfindungsgemäßen Verwendungen als auch auf das erfindungsgemäße Kosmetikum.

Es ist erfindungsgemäß vorteilhaft, wenn die erfindungsgemäße Zubereitung das 4-(tert.-Butyl)-4'-methoxydibenzoylmethan in einer Konzentration von 3 bis 5 Gewichts-%, bezogen auf das Gesamtgewicht der Zubereitung, enthält.

Es ist darüber hinaus erfindungsgemäß von Vorteil, wenn die erfindungsgemäße Zubereitung das 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) in einer Konzentration von 2 bis 4,5 Gewichts-%, bezogen auf das Gesamtgewicht der Zubereitung, enthält.

Erfindungsgemäß vorteilhafte Ausführungsformen der vorliegenden Erfindung sind auch dadurch gekennzeichnet, dass die Zubereitung 2-Phenylbenzimidazol-5-sulfonsäuresalze (insbesondere Na, K oder Ammoniumsalze) enthält. Diese werden erfindungsgemäß bevorzugt in einer Konzentration von 0,5-2,5 Gewichts-%, bezogen auf das Gesamtgewicht der Zubereitung eingesetzt.

Ferner ist es erfindungsgemäß von Vorteil, wenn die erfindungsgemäße Zubereitung Salicylsäure-2-ethylhexylester (INCI Octyl Salicylate) und/oder 2,4,6-Tris-[anilino-(p-carbo-2'-ethyl-1'-hexyloxy)]-1,3,5-triazin (INCI: Ethylhexyl Triazone) enthält.

Die erfindungsgemäß bevorzugte Einsatzkonzentration für Salicylsäure-2-ethylhexylester (INCI Octyl Salicylate) beträgt dabei 1,5-5 Gewichts-%, bezogen auf das Gesamtgewicht der Zubereitung.

Die erfindungsgemäß bevorzugte Einsatzkonzentration für 2,4,6-Tris-[anilino-(p-carbo-2'-ethyl-1'-hexyloxy)]-1,3,5-triazin (INCI: Ethylhexyl Triazone) beträgt 1,5-4,5 Gewichts-%, bezogen auf das Gesamtgewicht der Zubereitung.

Außerdem kann die erfindungsgemäße Zubereitung vorteilhaft Hexyl 2-[4-(diethylamino)-2-hydroxybenzoyl]benzoate (INCI: Diethylamino hydroxybenzoyl hexyl benzoate) und/oder 3,3,5-Trimethylcyclohexyl 2-hydroxybenzoat (INCI: Homosalate) enthalten.

Es ist erfindungsgemäß insbesondere von Vorteil, wenn die erfindungsgemäße Zubereitung Butylene Glycol Dicaprylate/Dicaprate enthält. Diese Ölkomponente wird erfindungsgemäß bevorzugt in einer Konzentration von 1,5-5 Gewichts-%, bezogen auf das Gesamtgewicht der Zubereitung eingesetzt.

Auch ist es erfindungsgemäß von Vorteil, wenn die erfindungsgemäße Zubereitung Cetearylalkohol enthält. Cetearylalkohol wird erfindungsgemäß bevorzugt in einer Konzentration von 0,5-3 Gewichts-%, bezogen auf das Gesamtgewicht der Zubereitung, eingesetzt.

Die erfindungsgemäße Zubereitung kann erfindungsgemäß in unterschiedlichen Formen vorliegen. Die erfindungsgemäß bevorzugten Formen sind dadurch gekennzeichnet, dass die Zubereitung in Form einer O/W-Emulsion vorliegt.

In einem solchen Fall ist es insbesondere erfindungsgemäß von Vorteil, wenn die Zubereitung Natriumstearylglutamat als Emulgator enthält.

Erfindungsgemäß vorteilhafte Ausführungsformen der vorliegenden Erfindung sind auch dadurch gekennzeichnet, dass die Zubereitung Di-n-butyladipat, Dicaprylylcarbonat, Propylheptylcaprylat und/oder C12-C15 Alkylbenzoat enthält.

Es ist erfindungsgemäß von Vorteil, wenn die erfindungsgemäße Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthält.

Erfindungsgemäß vorteilhafte Ausführungsformen der vorliegenden Erfindung sind auch dadurch gekennzeichnet, dass die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält.

Ferner ist es erfindungsgemäß von Vorteil, wenn die erfindungsgemäße Zubereitung Ethanol, Phenoxyethanol und/oder Ethylhexylglycerin enthält.

Nicht zuletzt sind erfindungsgemäß vorteilhafte Ausführungsformen dadurch gekennzeichnet, dass die Zubereitungen frei sind von Parabenen, Methylisothiazolinon, Chlormethylisothiazolinon und DMDM-Hydantoin.

Darüber hinaus können die erfindungsgemäßen Zubereitungen die für kosmetische Sonnenschutzmittel üblichen Inhaltsstoffe in den üblichen Einsatzkonzentrationen enthalten.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen. Diese Zubereitungen weisen ein nur geringes Augenreizungspotential Augenirritationspotential auf.

| | **1** | **2** | **3** | **4** |
|---|---|---|---|---|
| **INCI** | **m[%]** | **m[%]** | **m[%]** | **m[%]** |
| Butyl Methoxydibenzoylmethane | 3,00 | 3,00 | 5,00 | 4,00 |
| Phenylbenzimidazole Sulfonic Acid | 1,00 | 1,00 | 1,50 | 2,50 |
| Ethylhexyl Salicylate | 2,00 | 4,00 | 4,50 | 4,00 |
| Ethylhexyl Triazone | 1,50 | 2,00 | 3,00 | 3,00 |
| Homosalate | | 8,00 | 2,00 | 3,00 |
| Bis-Ethylhexyloxyphenol Methoxyphenyl Triazine | 2,00 | 4,00 | 3,00 | 3,00 |
| Diethylamino Hydroxybenzoyl Hexyl Benzoate | | 1,00 | | |
| Titanium Dioxide | 0,5 | | | |
| Trisodium EDTA | 0,20 | 0,20 | 0,20 | 0,20 |
| Aqua | ad 100 | ad 100 | ad 100 | ad 100 |
| Alcohol Denat. | 4,00 | | 5,00 | 3,00 |
| Cetearyl Alcohol | 2,00 | 1,50 | 1,00 | 1,50 |
| Behenyl Alcohol | | | 1,00 | 1,50 |
| Xanthan Gum | 0,10 | | 0,10 | 0,05 |
| Acrylates/C10-30 Alkyl Acrylate Crosspolymer | 0,15 | | | 0,10 |
| Carbomer | 0,10 | | 0,10 | 0,20 |
| Methylparaben | | 0,20 | | |
| Phenoxyethanol | 0,60 | 0,80 | 0,90 | 0,70 |
| Sodium Hydroxide | 0,40 | 0,15 | 0,30 | 0,50 |
| Glycerin | 6,00 | 1,00 | 1,00 | 5,00 |
| 1,2-Hexanediol | 0,5 | | | |
| Methylpropanediol | | 1,00 | | 2,00 |
| Parfum | 0,30 | 0,30 | 0,30 | 0,30 |
| Silica Dimethyl Silylate | | 0,50 | 0,50 | |
| Distarch Phosphate | 2,00 | 1,00 | 1,00 | |
| Tapioca Starch | | | | 1,00 |
| Silica | | | 1,00 | 2,00 |
| Glyceryl Stearate | 0,80 | | | |
| Sodium Cetearyl Sulfate | | 0,20 | | |
| Glyceryl Stearate SE | | 2,50 | | |
| Sodium Stearoyl Glutamate | 0,40 | | 0,30 | 0,50 |
| Dibutyl Adipate | 2,00 | | | 3,00 |
| Dimethicone | 0,80 | | 0,50 | 1,00 |
| Butylene Glycol Dicaprylate/Dicaprate | 3,00 | 3,00 | 5,00 | 3,50 |
| Dicaprylyl Carbonate | | | | 1 |
| Cyclomethicone | | | 1,00 | 1,00 |
| Propylheptyl Caprylate | | | 1,00 | |
| C12-15 Alkyl Benzoate | 4,00 | 3,00 | | 2,00 |
| CI 77492 + CI 77491 + CI 77499 | | | 0,20 | |
| CI 77492 | | | 0,30 | |
| CI 77891 | | | 3,00 | |
| CI 77491 | | | 0,05 | |
| CI 77491 + CI 77499 | | | 0,10 | |
| Ethyl hexylglycerin | 0,30 | | | 0,20 |
| Ubiquinone | 0,03 | | | |
| Glycyrrhiza Inflata Root Extract | 0,03 | 0,03 | | |
| Panthenol | | | 1,00 | |
| Tocopheryl Acetate | 0,10 | 0,10 | 0,50 | 0,50 |

## Patentansprüche

1. Verwendung einer UV-Filterkombination enthaltend 4-(tert.-Butyl)-4'-methoxydibenzoylmethan und 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) in kosmetischen Sonnenschutzmitteln, zur Verhinderung von Augenirritationen, wobei das Sonnenschutzmittel frei ist von 3-(4-Methylbenzyliden)-campher, 2-Hydroxy-4-methoxybenzophenon (INCI: Oxybenzon), Ethylhexyl-2-cyano-3,3-diphenylacrylat (INCI: Octocrylen) und 4-Methoxyzimtsäure-2-ethylhexylester (INCI Octylmethoxycinnamate).

2. Verwendung einer UV-Filterkombination enthaltend 4-(tert.-Butyl)-4'-methoxydibenzoylmethan und 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) in kosmetischen Sonnenschutzmitteln, um das Einfließen desselben ins Auge bei der Anwendung im Gesicht zu verhindern, wobei das Sonnenschutzmittel frei ist von 3-(4-Methylbenzyliden)-campher, 2-Hydroxy-4-methoxybenzophenon (INCI: Oxybenzon), Ethylhexyl-2-cyano-3,3-diphenylacrylat (INCI: Octocrylen) und 4-Methoxyzimtsäure-2-ethylhexylester (INCI Octylmethoxycinnamate).

3. Kosmetikum aus
a) einem Sonnenschutzmittel enthaltend 4-(tert.-Butyl)-4'-methoxydibenzoylmethan und 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine), wobei das Sonnenschutzmittel frei ist von 3-(4-Methylbenzyliden)-campher, 2-Hydroxy-4-methoxybenzophenon (INCI: Oxybenzon), Ethylhexyl-2-cyano-3,3-diphenylacrylat (INCI: Octocrylen) und 4-Methoxyzimtsäure-2-ethylhexylester (INCI Octylmethoxycinnamate), und
b) einem Packmittel, wobei das Packmittel einen schriftlichen Hinweis darauf enthält, dass das Sonnenschutzmittel ein nur geringes Augenreizungspotential aufweist.

4. Verwendung nach einem der Ansprüche 1 und 2 oder Kosmetikum nach Anspruch 3, **dadurch gekennzeichnet, dass** die Zubereitung das 4-(tert.-Butyl)-4'-methoxydibenzoylmethan in einer Konzentration von 3 bis 5 Gewichts-%, bezogen auf das Gesamtgewicht der Zubereitung, enthält.

5. Verwendung oder Kosmetikum nach einem der vorhergehenden Ansprüche, **dadurch gekennzeichnet, dass** die Zubereitung das 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) in einer Konzentration von 2 bis 4,5 Gewichts-%, bezogen auf das Gesamtgewicht der Zubereitung, enthält.

6. Verwendung oder Kosmetikum nach einem der vorhergehenden Ansprüche, **dadurch gekennzeichnet, dass** die Zubereitung 2-Phenylbenzimidazol-5-sulfonsäuresalze enthält.

7. Verwendung oder Kosmetikum nach einem der vorhergehenden Ansprüche, **dadurch gekennzeichnet, dass** die Zubereitung Salicylsäure-2-ethylhexylester (INCI Octyl Salicylate) und/oder 2,4,6-Tris-[anilino-(p-carbo-2'-ethyl-1'-hexyloxy)]-1,3,5-triazin (INCI: Ethylhexyl Triazone) enthält.

8. Verwendung oder Kosmetikum nach einem der vorhergehenden Ansprüche, **dadurch gekennzeichnet, dass** die Zubereitung Hexyl 2-[4-(diethylamino)-2-hydroxybenzoyl]benzoate (INCI: Diethylamino hydroxybenzoyl hexyl benzoate) und/oder 3,3,5-Trimethylcyclohexyl 2-hydroxybenzoat (INCI: Homosalate) enthält.

9. Verwendung oder Kosmetikum nach einem der vorhergehenden Ansprüche, **dadurch gekennzeichnet, dass** die Zubereitung Butylene Glycol Dicaprylate/Dicaprate enthält.

10. Verwendung oder Kosmetikum nach einem der vorhergehenden Ansprüche, **dadurch gekennzeichnet, dass** die Zubereitung Cetearylalkohol enthält.

11. Verwendung oder Kosmetikum nach einem der vorhergehenden Ansprüche, **dadurch gekennzeichnet, dass** die Zubereitung in Form einer O/W-Emulsion vorliegt.

12. Verwendung oder Kosmetikum nach einem der vorhergehenden Ansprüche, **dadurch gekennzeichnet, dass** die Zubereitung Natriumstearylglutamat als Emulgator enthält.

13. Verwendung oder Kosmetikum nach einem der vorhergehenden Ansprüche, **dadurch gekennzeichnet, dass** die Zubereitung Di-n-butyladipat, Dicaprylylcarbonat, Propylheptylcaprylat und/oder C12-C15 Alkylbenzoat enthält.

14. Verwendung oder Kosmetikum nach einem der vorhergehenden Ansprüche, **dadurch gekennzeichnet, dass** die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthält.

15. Verwendung oder Kosmetikum nach einem der vorhergehenden Ansprüche, **dadurch gekennzeichnet, dass** die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält.

16. Verwendung oder Kosmetikum nach einem der vorhergehenden Ansprüche, **dadurch gekennzeichnet, dass** die Zubereitung Ethanol, Phenoxyethanol und/oder Ethylhexylglycerin enthält.

17. Verwendung oder Kosmetikum nach einem der vorhergehenden Ansprüche, **dadurch gekennzeichnet, dass** die Zubereitung frei ist von Parabenen, Methylisothiazolinon, Chlormethylisothiazolinon und DMDM-Hydantoin.
